# EUROPEAN PATENT APPLICATION

(11) **EP 4 517 795 A1**
(43) Date of publication of application: **05.03.2025**
(21) Application number: 23796335.0
(22) Date of filing: 24.04.2023
(51) Int. Cl.: H01J 37/305, B82Y 40/00, G01N 23/04, G01N 33/483, G01N 27/00

(54) **METHOD AND DEVICE FOR PRODUCING NANOPORES**

(30) Priority: 28.04.2022 JP 2022074270
(71) Applicant: The University of Tokyo, Bunkyo-ku, Tokyo 113-8654 (JP)
(72) Inventor: HSU, Wei-Lun, Tokyo 113-8654 (JP); TAKAMOTO, Akihide, Tokyo 113-8654 (JP); OSHIKAWA, Hiroyuki, Tokyo 113-8654 (JP); MARUYAMA, Shigeo, Tokyo 113-8654 (JP); DAIGUJI, Hirofumi, Tokyo 113-8654 (JP); LEE, Chun Yen, Tokyo 113-8654 (JP)
(74) Representative: Michalski Hüttermann & Partner Patentanwälte mbB
(86) International application number: PCT/JP2023/016155
(87) International publication number: WO 2023/210595

(57) **Abstract**

One embodiment provides a method for processing a thin film, the method including (a) providing a thin film; (b) irradiating the thin film with an electron beam; (c) observing the thin film to determine whether a pore satisfying a predetermined condition has been formed; and (d) if it is determined in (c) that the pore satisfying the predetermined condition has not been formed, repeating (b) and (c).

## Description

### TECHNICAL FIELD

The present disclosure relates to a method and a device for producing a nanopore.

### BACKGROUND ART

Nanometer-sized pores (nanopores) formed in a thin film of a solid material have been demonstrated to be useful for sensing a substance passing therethrough and used in various applications. For example, a nucleotide sequence can be determined by causing a nucleic acid (DNA or RNA) to pass through a nanopore and measuring a change in the electrical signal at that time.

When a thin film having a nanopore is immersed in a conducting fluid and a voltage potential is applied between both sides thereof, an ionic current passing through the nanopore can be observed. This ionic current is determined by the effective cross-sectional area of the nanopore. When a nucleic acid molecule passes through a nanopore, the molecule partially clogs the nanopore to change the magnitude of the current passing through the nanopore. Since each nucleotide has a specific shape and size, the ionic current changes depending on individual nucleotides. The sequence of nucleotides can be determined by reading this change.

Thus, nanopore-sensing is sensitive to the size (diameter) and the shape of a nanopore.
Therefore, a process for producing a nanopore in a thin film needs to be controlled at the nanometer level.

The thickness of the thin film affects the sensitivity. A nucleotide has a nanometer-level size in the moving direction. In a case of a large film thickness, a plurality of nucleotides is present inside a nanopore in the thickness direction of the thin film at a certain point of time. Accordingly, use of a nanometer-sized thin film improves the reading accuracy.

It has also been reported that nanopores can be formed in a MoS₂ monolayer film having a small and uniform thickness using a transmission electron microscope (TEM) or an electrochemical reaction (ECR) (NPL 1).

However, a thin film having a nanometer-level thickness is sensitive to effects caused in the production process. For example, such a thin film is deformed or damaged by a mechanical or thermal stress or impact due to a lithographic process, electron beam irradiation, and the like. For example, in a case of electron beam irradiation, although the size of a nanopore can be controlled by the irradiation time, there is a problem in controlling the size and the shape.

To solve these problems, there is a need of a technique of forming a nanopore in a solid thin film having a nanometer-level thickness with high precision.

### CITATION LIST

### NON PATENT LITERATURE

NPL 1: Graf, M., Lihter, M., Thakur, M. et al., Fabrication and practical applications of molybdenum disulfide nanopores, Nat Protoc 14, 1130-1168 (2019)

### SUMMARY OF INVENTION

An aspect of the present disclosure provides a method for processing a thin film, the method including (a) providing a thin film; (b) irradiating the thin film with an electron beam; (c) observing the thin film to determine whether a pore satisfying a predetermined condition has been formed; and (d) if it is determined in (c) that the pore satisfying the predetermined condition has not been formed, repeating (b) and (c).

Further aspects and advantages of the present disclosure will become readily apparent to those skilled in this art on the basis of the following detailed description, where only exemplary embodiments of the present disclosure are disclosed and described. As will be understood, other different embodiments are possible in the present disclosure, and some details thereof can be modified in various, obvious points without departing from the present disclosure. Accordingly, the drawings and description are to be regarded as illustrative in nature, and not as restrictive.

### BRIEF DESCRIPTION OF DRAWINGS

[Fig. 1] Fig. 1 illustrates a flowchart of a method for processing a thin film according to an embodiment of the present disclosure.
[Fig. 2] Fig. 2 illustrates a flowchart of a method for processing a thin film according to an embodiment of the present disclosure.
[Fig. 3] Fig. 3 illustrates a flowchart of a method for processing a thin film according to an embodiment of the present disclosure.
[Fig. 4] Fig. 4 includes schematic views of the vicinity of a pore for explaining correction of a pore shape according to an embodiment of the present disclosure.
[Fig. 5] Fig. 5 includes Ronchigram images showing the formation of a nanopore in an Example.
[Fig. 6] Fig. 6 includes electron microscope images of a nanopore produced in an Example.
[Fig. 7] Fig. 7 includes electron microscope images of a nanopore produced in an Example.
[Fig. 8] Fig. 8 illustrates a schematic view for explaining the configuration of a nanopore processing system according to an embodiment of the present disclosure.

### DESCRIPTION OF EMBODIMENTS

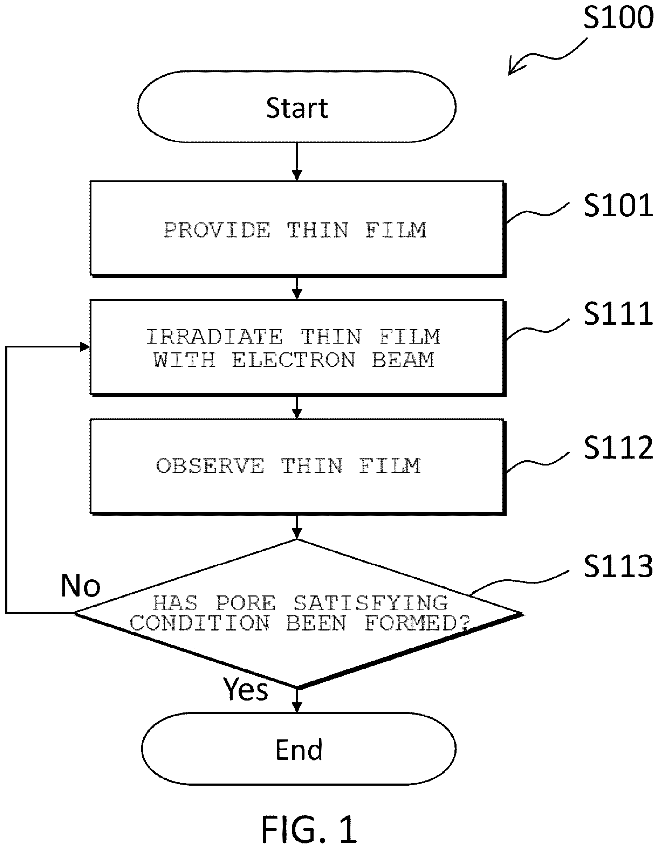

Fig. 1 illustrates a flowchart S100 of a method for processing a thin film according to an embodiment of the present disclosure. In Step S101, a thin film is provided. In Step S111, the thin film is irradiated with an electron beam. In Step S112, an electron beam-irradiated region of the thin film is observed. In S113, whether a nanopore satisfying a predetermined condition has been formed in the thin film is determined. If it is determined that a predetermined nanopore has been formed, the process is terminated. If it is determined that a predetermined nanopore has not been formed, Steps S111 and S112 are repeated. Specifically, substantially the same region is again irradiated with the electron beam (S111), the region irradiated with the electron beam is observed (S112), and whether a nanopore satisfying the predetermined condition has been formed in the thin film is determined (S113). This is repeated until it is determined that a predetermined nanopore has been formed.

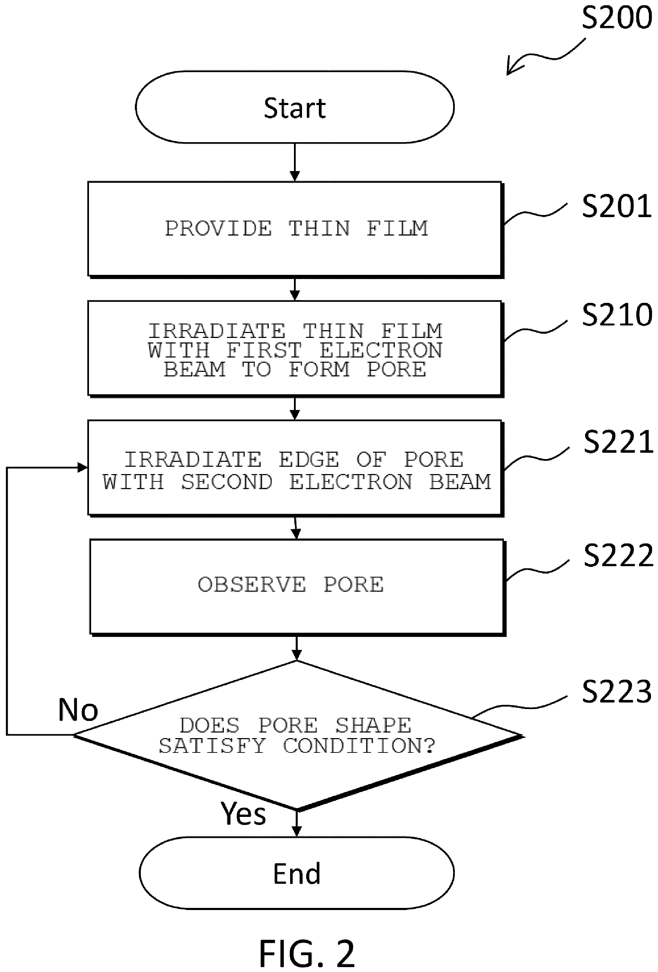

Fig. 2 illustrates a flowchart S200 of a method for processing a thin film according to an embodiment of the present disclosure. In Step S201, a thin film is provided. In Step S210, the thin film is irradiated with a first electron beam to form a nanopore. Thus, a through hole is formed in an irradiation region for the first time. In Step S221, a thin-film region in the vicinity of the nanopore or a thin-film region on an edge of the pore is irradiated with a second electron beam. In Step S222, the shape of the nanopore is observed. In S223, whether the shape of the nanopore satisfies a predetermined condition is determined. If it is determined that the shape of the nanopore satisfies the predetermined condition, the process is terminated. If it is determined that the shape of the nanopore does not satisfy the predetermined condition, Steps S221 and S222 are repeated. Specifically, the edge of the pore is again irradiated with the second electron beam (S221), the region irradiated with the electron beam is observed (S222), and whether the shape of the nanopore satisfies the predetermined condition is determined (S223). This is repeated until it is determined that the shape of the nanopore satisfies the predetermined condition. The irradiation region may be changed each time of repetition. This enables, for example, better adjustment of the shape.

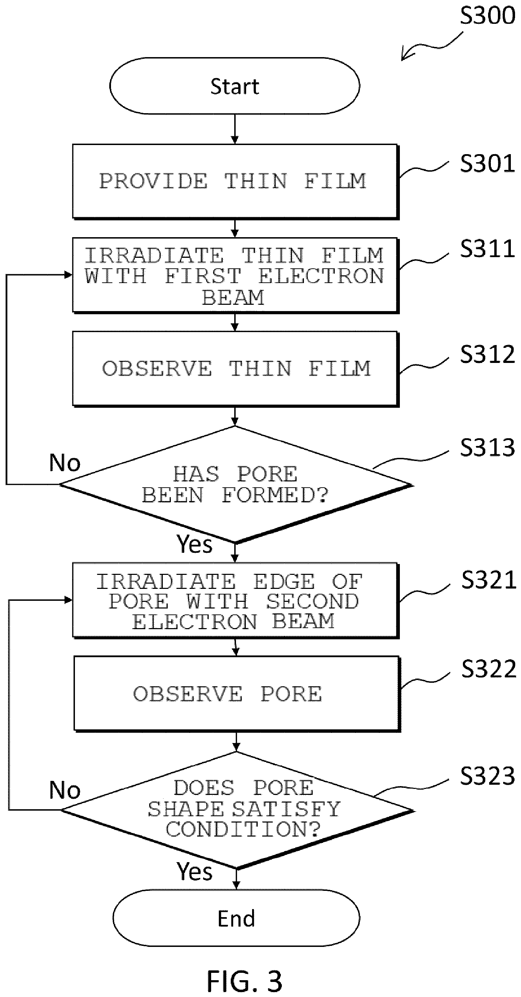

Fig. 3 illustrates a flowchart S300 of a method for processing a thin film according to an embodiment of the present disclosure. In Step S301, a thin film is provided.

In Step S311, a region of the thin film where a nanopore is to be formed is irradiated with a first electron beam. In Step S312, the electron beam-irradiated region of the thin film is observed. In S313, whether a nanopore has been formed in the thin film is determined. If it is determined that a nanopore has not been formed, Steps S311 and S312 are repeated. Specifically, substantially the same region is again irradiated with the first electron beam (S311), the region irradiated with the electron beam is observed (S312), and whether a nanopore has been formed in the thin film is determined (S313). This is repeated until it is determined that a predetermined nanopore has been formed. Thus, a through hole is formed in the irradiation region for the first time. In addition, the through hole formed for the first time can also have a good shape.

In some embodiments, if the formed nanopore satisfies a predetermined condition, the process may be terminated (this step is not illustrated). Alternatively, as illustrated in Fig. 3, adjustment of the pore shape (Steps S321 to S323) may be performed.

In Step S321, a thin-film region in the vicinity of the nanopore or a thin-film region on an edge of the pore is irradiated with a second electron beam. In Step S322, the shape of the nanopore is observed. In S323, whether the shape of the nanopore satisfies the predetermined condition is determined. If it is determined that the shape of the nanopore satisfies the predetermined condition, the process is terminated. If it is determined that the shape of the nanopore does not satisfy the predetermined condition, Steps S321 and S322 are repeated. Specifically, the edge of the pore is again irradiated with the second electron beam (S321), the region irradiated with the electron beam is observed (S322), and whether the shape of the nanopore satisfies the predetermined condition is determined (S323). This is repeated until it is determined that the shape of the nanopore satisfies the predetermined condition. The irradiation region may be changed each time of repetition. This enables, for example, better adjustment of the shape.

In some embodiments, the first electron beam used for forming a pore for the first time and the second electron beam used for adjusting the shape of the pore may be electron beams under the same conditions. In some embodiments, the first electron beam used for forming a pore for the first time and the second electron beam used for adjusting the shape of the pore may be electron beams under conditions different from each other.

### <Thin film>

The "thin film" as also used herein generally refers to a material or a component formed as a film at least in a region including a pore and the vicinity of the pore and having a film thickness that is sufficiently larger than the size of the pore in the film plane direction. The thin film may be a so-called two-dimensional (2D) material. In some embodiments, the thin film may contain an inorganic material. In some embodiments, the thin film may contain an organic material. In some embodiments, the thin film may be formed of substantially a single material. In some embodiments, the thin film may be formed of a plurality of materials, layers, or members.

Examples of the material of the thin film include, but are not limited to, graphene family, Xenes (groups IV, V, and VI graphene-like two-dimensional materials), chalcogenides, and oxides.

The chalcogenides may be transition metal chalcogenides. The chalcogenides may be any of monochalcogenides (MX), dichalcogenides (MX₂, TMDC), trichalcogenides (MX₃), and tetrachalcogenides (MX₄). M represents a transition metal such as molybdenum (Mo), tungsten (W), niobium (Nb), or tantalum (Ta), and X represents a chalcogen atom selected from sulfur (S), selenium (Se), and tellurium (Te). The chalcogenides may be topological insulators such as Bi₂Te₃, Bi₂Se₃, and Sb₂Te₃.

The thin film may be a monoatomic layer (two-dimensional material) of a chalcogenide such as MoS₂. The thin film may have a monoatomic layer in at least a pore region. The thin film may be, for example, a monoatomic layer of a chalcogenide such as MoS₂. Such a monolayer film can be produced by mechanical exfoliation (ME) from a bulk crystal with Scotch tape, chemical vapor deposition (CVD), mist CVD, molecular-beam epitaxy (MBE), or another known or new method.

In some embodiments, for example, the number of atomic layers in a target region and the fact that the target region of the thin film is a monoatomic layer can be measured or checked by Raman spectroscopy. The thickness of the thin film can be measured or checked using, for example and without limitation, a transmission electron microscope (TEM), an atomic force microscope (AFM), an optical microscope, or the like.

### <Electron beam>

An electron beam may be generated by an electron microscope. The electron beam may be generated using a transmission electron microscope (TEM), a scanning electron microscope (SEM), a scanning transmission electron microscope (STEM), or another electron gun and guided to the thin film. The electron beam may be a focused electron beam.

The electron beam may have a beam diameter smaller than a desired pore size (diameter or the minimum diameter) when irradiating the thin film. The diameter of the electron beam may be defined by a so-called spot size of an electron microscope. Another definition may be used for the diameter of the electron beam. The diameter of the electron beam may be, for example, 50 nm, 30 nm, 25 nm, 20 nm, 15 nm, 10 nm, 9 nm, 8 nm, 7 nm, 6 nm, 5 nm, 4 nm, 3 nm, 2.8 nm, 2.6 nm, 2.5 nm, 2.4 nm, 2.3 nm, 2.2 nm, 2.1 nm, 2.0 nm, 1.9 nm, 1.8 nm, 1.7 nm, 1.6 nm, 1.5 nm, 1.4 nm, 1.3 nm, 1.2 nm, 1.1 nm, 1.0 nm, 0.9 nm, 0.8 nm, 0.7 nm, 0.6 nm, 0.5 nm, 0.4 nm, or 0.3 nm.

The accelerating voltage of an electron beam source may be, for example, 200 kV, 160 kV, 120 kV, 100 kV, 80 kV, 60 kV, 50 kV, 40 kV, 30 kV, 20 kV, 10 kV, 9 kV, 8 kV, 7 kV, 6 kV, 5 kV, 4 kV, 3 kV, 2 kV, or 1 kV.

The current of the electron beam is, for example and without limitation, 0.5 nA (1 nm probe).

In the present disclosure, irradiation with an electron beam is mainly described. However, in some embodiments, other energy beams may be used to irradiate the thin film. For example, particle beams (such as an ion beam, a focused ion beam, and a focused atomic beam) may be used.

### <Pore>

A "pore" (also referred to as an aperture or a through hole) as used herein generally refers to a through hole formed in the thickness direction of a thin film. In "irradiating an edge of a pore", irradiation is performed on an area within the thin film in the vicinity of a pore, and the irradiation region is not a location away from the pore. It does not mean irradiating the inside of the pore but means that irradiation is performed so as to be geometrically or substantially in contact with or overlap the pore.

### <Repeated irradiation and observation of nanopore>

Atoms, a group of atoms, molecules, or a group of molecules (hereinafter, simply referred to as "atoms") of the thin film may be removed or etched by irradiation with an electron beam. Atoms of the thin film are ejected from the thin film by the kinetic energy of electrons or heat due to electron irradiation. Thus, the thin film can be scraped off, a pore can be opened after a sufficient irradiation time, and/or atoms in the thin film in the vicinity of the pore can be removed. However, the mechanism of the removal of thin film atoms is not limited to the above, and other or different explanations are possible.

Some embodiments may include continuous irradiation with an electron beam under a certain condition to form a pore in a thin film or to observe the shape of the thin film. In some embodiments, observation and pore formation may be performed at the same time. For example, when observation is performed by electron beam irradiation (for example, in a scanning mode), the electron beam irradiation causes pore formation or a change in the pore shape.

Some embodiments may include irradiating a thin film with a (focused or unfocused) electron beam a plurality of times to form a pore in the thin film or to form the shape of a thin film. For example, the focal point of a focused electron beam may be moved in the thickness direction of the thin film. When the focal point lies within the thin film or immediately before or after that, the thin film is substantially processed by the electron beam. When the focal point lies outside the thin film, the thin film is not processed by the electron beam.

In some embodiments, for a predetermined position of a predetermined thin film, electron beam irradiation and observation of the field of view including the irradiation region may be repeated. Conditions for each electron beam irradiation (such as the time of irradiation, the current and voltage of the electron beam, the electron beam diameter or the spot size, and the convergence angle) may be set such that one electron beam irradiation alone does not open a pore or does not substantially change the shape of a pore that has already been formed. For example, a time (tₚₒᵣₑ) necessary for opening a pore or changing the shape of a pore is determined in advance, and the time for each electron beam irradiation (with other conditions set to be the same) (tᵢᵣᵣ) may be set to be shorter than (tᵢᵣᵣ < tₚₒᵣₑ) or sufficiently shorter (tᵢᵣᵣ << tₚₒᵣₑ) than the time. The time for each electron beam irradiation (tᵢᵣᵣ) may be set such that a pore can be opened or the shape of a pore can be changed for the first time by repeating the electron beam irradiation a plurality of times (N ≥ 2). (N × tᵢᵣᵣ > tₚₒᵣₑ > (N - 1) × tᵢᵣᵣ)

The number of times (N) of electron beam irradiation necessary for opening a pore or changing the shape of a pore can vary, even for the same thin-film material, depending on the thickness thereof and other conditions. Accordingly, the time (tₚₒᵣₑ) necessary for opening a pore or changing the shape of a pore cannot be determined in advance in some cases. Therefore, by repeating electron beam irradiation a plurality of times under sufficiently weak electron beam irradiation conditions, damage to the thin film can be reduced to the minimum and/or a pore having a smaller size and a more well-formed shape can be formed.

**In** some embodiments, between any of two electron beam irradiations, an image of the irradiation region may be acquired to check the formation or deformation of a pore. After each time of electron beam irradiation, an image of the irradiation region may be acquired to check the formation or deformation of a pore.

**In** the case of using a STEM, the image of the irradiation region may be acquired, for example, in a spot irradiation mode in a defocused state. **In** some embodiments, a thin film may be processed when a focused electron beam is focused on the thin film in the spot irradiation mode of a STEM, and an image of the irradiation region may be acquired and/or observed when the focused electron beam is defocused from the thin film. This enables processing of the thin film and observation of the irradiation region to be efficiently performed. Although a defocused image in the spot irradiation mode may be unclear compared with a bright-field image, such a defocused image has a sufficient quality to determine whether a pore is opened or not and/or the shape of a pore is changed or not.

**In** some embodiments, an image of the irradiation region may be acquired as, for example, a bright-field image or a dark-field image.

The "observing a thin film" as used herein may include acquiring and analyzing an image of a thin film and may include measuring a certain property of a thin film.

### <Shape of pore>

In some embodiments, to determine the shape of a pore, for example, the pore may be approximated by an ellipse, and the shape of the pore may be expressed by the major axis and the minor axis of the ellipse. For example, the shape of the pore may be expressed by using two diameters orthogonal to each other. For example, the maximum diameter of the pore and a length (for example, the minimum value or the maximum value) of a diameter in a direction nonparallel (for example, orthogonal) to the maximum diameter may be used. The size of the pore may be determined by subjecting a microscope image or the like to image processing. For example, image processing software may be used to calculate an average diameter based on the area of the pore or to calculate values of other geometrical parameters.

The shape adjustment of the pore may be repeated until the shape falls within a predetermined condition. For example, a ratio of the minor axis to the major axis (ellipticity) of an ellipse approximating the pore may be used. The condition for terminating pore processing may be that the ratio of the minor axis to the major axis (ellipticity) is a value such as 0.5, 0.6, 0.7, 0.8, 0.85, 0.9, or 0.95, or a larger value. The condition for terminating pore processing may be that a ratio of the difference between the major axis and the minor axis of the pore and the average diameter is a value such as 0.05, 0.1, 0.2, 0.3, 0.4, or 0.5, or a smaller value.

### <Adjustment of pore shape>

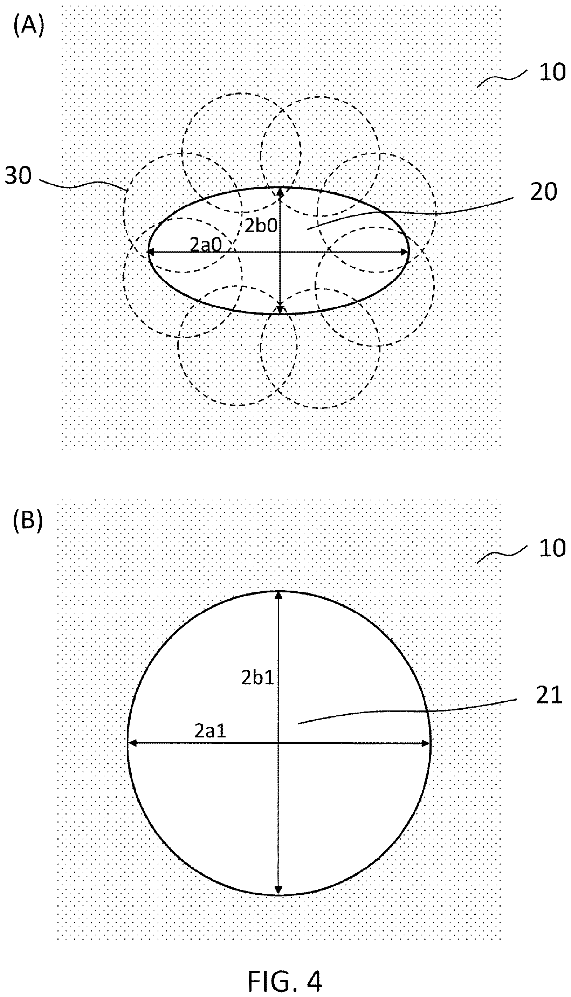

A method for adjusting the shape of a pore that has once been formed will be described with reference to Fig. 4. Fig. 4 schematically illustrates real images of a thin film having a pore. As illustrated in Fig. 4(A), an initial through hole or pore 20 is formed in a thin film 10. However, the pore 20 does not have a desired shape (a perfect circle in the example in Fig. 4). In Fig. 4(A), the pore 20 is approximated by an ellipse as a simple example. The size of the pore 20 is long in one direction (major axis, length 2 × a0) and is short in the other direction (minor axis, length 2 × b0): a0 > b0.

In order to make the shape of the pore 20 a perfect circle, part of the thin film is removed mainly on the edge in the minor axis direction. An irradiation region 30 is moved, and the thin film at each positon is removed. Also in this removal, focused-defocused irradiation may be performed (Fig. 4(A)). This enables mild removal of each portion and processing with high precision. Performing such irradiation in sequence can mainly increase the minor axis.

As a result, as illustrated in Fig. 4(B), a nanopore close to a desired perfect circular shape can be realized after shape adjustment. Major axis: 2 × a1, Minor axis: 2 × b1 (a1 ≈ b1).

As for the condition for a perfect circle, for example, a predetermined ratio of the minor axis to the major axis (ellipticity) is specified, and processing may be completed on the condition that the ratio becomes larger than this ellipticity. The condition for a perfect circle may be specified using, for example, a predetermined circularity.

### <Example 1>

### <1 Preparation of MoS₂ monolayer crystal>

A low-tack adhesive tape was attached to a bulk MoS₂ single-crystal material, and a surface layer of the material was transferred to the low-tack adhesive tape by mechanical exfoliation (ME). Furthermore, this was attached to another low-tack adhesive tape and peeled off, and these operations were repeatedly performed to cause cleavage. Thus, a MoS₂ monolayer crystal was generated on an adhesive surface of a low-tack adhesive tape in the end. The MoS₂ monolayer crystal was transferred to a polydimethylsiloxane (PDMS) supporting material formed on a cover glass.

It was confirmed by Raman spectroscopy that the obtained MoS₂ thin film was a monolayer crystal. Specifically, in the MoS₂ thin film used in this Example, a peak (A_{1g} peak) of the Raman spectrum due to vibrations of MoS₂ atoms in the interlayer direction, and a peak (E¹_{2g} peak) due to vibrations in the plane direction were observed at 403.092 cm⁻¹ and 304.316 cm⁻¹, respectively. This confirmed that this MoS₂ thin film was a monolayer crystal. It was also confirmed by the R value of a channel image of an optical microscope, which is linearly related to the number of layers, that the obtained MoS₂ thin film was a monolayer crystal. Specifically, the R value of an optical microscope (DSX100, manufactured by Olympus Corporation) was 25 to 30 (exposure time: 50 mS, ISO value: 200), and this confirmed that this MoS₂ thin film was a monolayer crystal.

A free-standing Si₃N₄ film supported on a silicon chip was prepared as a support material, and a 500 nm through hole was formed in the support material by focused ion beam (FIB). The thin film of the MoS₂ monolayer crystal obtained as described above was transferred to the surface of the Si₃N₄ film so as to cover the through hole, and the PDMS was removed.

### <2 Conditions for STEM>

In this Example, a STEM (JEM-ARM200F, JEOL Ltd.) was used. This STEM can be switched between a scanning mode and a Ronchigram mode. The accelerating voltage is 200 kV. A probe having the smallest size was used, and the aperture was set to the minimum. Thus, damage to regions other than a target region was reduced.

If focusing is performed in the target region, damage near a nanopore increases. Furthermore, the accuracy of characteristics of the nanopore, such as a predetermined size and shape, may decrease. Accordingly, in order to reduce damage to the target region by electron beam irradiation, STEM focal point setting may be performed in advance in the target region.

For example, focusing may be performed on a support material such as a Si₃N₄ film, and an electron beam may then be moved to the target region. For example, focusing may be performed at a position of a support material in a region where a thin film such as a MoS₂ monolayer crystal is not supported, and an electron beam may then be moved to the target region of the thin film. For example, focusing may be performed at a position of a support material in a region where a thin film such as a MoS₂ monolayer layer crystal is not supported, next, focusing may be performed at a position of the support material in a region where the thin film such as a MoS₂ monolayer crystal is supported, and an electron beam may then be moved to the target region of the thin film. Focusing at a plurality of positions before irradiation of the target region can shorten the electron beam irradiation time for focusing in the target region.

For each focusing, various methods can be employed. Focusing may be performed on the basis of, for example and without limitation, a Ronchigram, a lattice image, a diffraction image, a diffraction contrast, or the like.

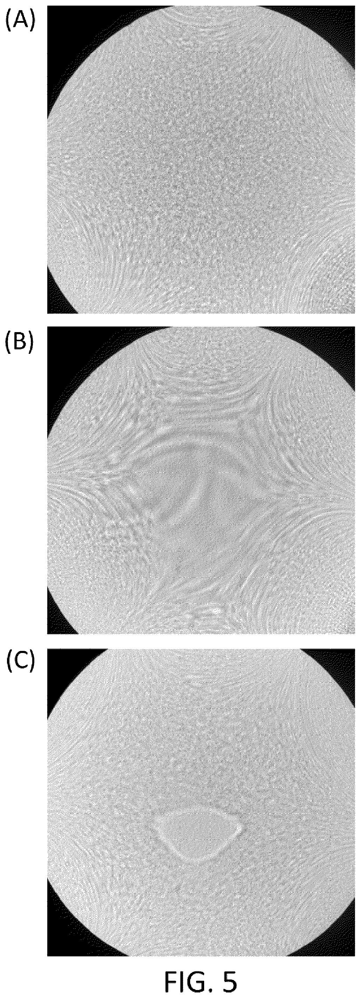

The MoS₂ monolayer crystal was under-focused, and the magnification was set to × 20M or more. The mode was changed from the scanning mode to the Ronchigram mode. A typical image at that time is shown in Fig. 5(A). Fig. 5(A) shows an image obtained with 500 nm under-focus.

The MoS₂ monolayer film was focused at a position where a pore was to be formed, and the focus was kept so that the Ronchigram was visible. A typical Ronchigram image at that time is shown in Fig. 5(B).

The focal point was returned to under-focus, and the formation of the pore was checked. A typical image at that time is shown in Fig. 5(C). Fig. 5(C) shows an image obtained with 500 nm under-focus.

The checking of the pore may be performed when an image in a central portion of the Ronchigram has changed or disappeared. The checking of the pore may be performed in the scanning mode or the Ronchigram mode.

In some embodiments, the checking of the pore may be performed in the scanning mode. In some embodiments, focus and defocus (under-focus or over-focus) may be alternated in the state of the Ronchigram mode without switching the mode. The time taken for mode switching can be saved, and damage to the thin film can be reduced. In other words, in the Ronchigram mode, the focal point is moved in the Z direction so as to pass through the thin film. The thin film is substantially sputtered when the focal point lies on the thin film. By observing an image during defocus, whether a first pore has been formed or not can be checked.

### <3 Shape adjustment of pore>

### <Shape adjustment of pore-1>

In some embodiments, electron beam irradiation and observation may be repeatedly performed until a pore has a predetermined size.

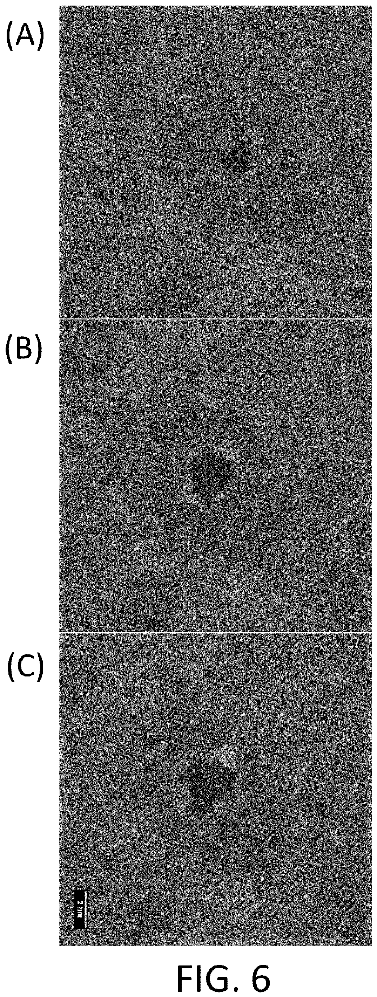

In some embodiments, if a first pore is formed, and the size of the pore does not reach a predetermined size, for example, observation of the pore is continued in the scanning mode. The size of the pore can be increased by electron beam irradiation for the observation. Fig. 6(A) shows a TEM image immediately after the formation of the first pore. After this, the observation was continued at a magnification of about × 10M to 20M. Fig. 6(B) shows a TEM image after five minutes of irradiation, and Fig. 6(C) shows a TEM image after seven minutes of irradiation. When the size of the pore has reached a desired size, the electron beam irradiation is stopped, and the observation is finished to finish the shape adjustment of the pore. The pore size can be accurately controlled by continuously performing electron beam irradiation through observation in this manner.

### <Shape adjustment of pore-2>

In some embodiments, irradiation may be performed while the position of electron beam irradiation is moved, and observation of a pore may be performed each time. Electron beam irradiation and observation may be repeatedly performed until the pore has a predetermined size.

In some embodiments, focused-defocused irradiation may be performed while the in-plane position of the irradiation is moved with respect to the edge of a nanopore that has once been formed such that the shape of the nanopore is close to a perfect circle.

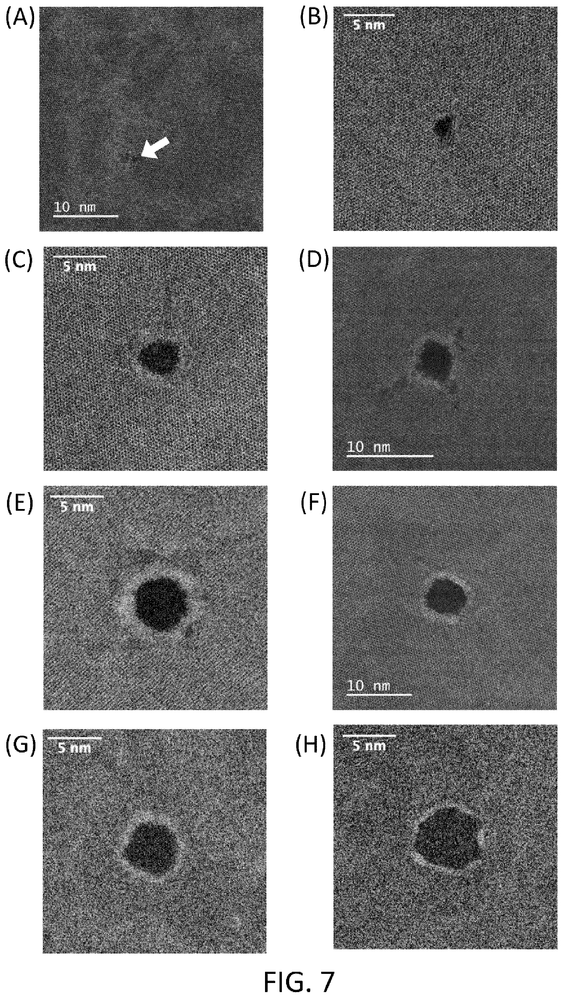

As one Example, nanopores having target diameters (d = 0.7 nm to 9 nm) were created. Figs. 7(A) to (H) show real images of STEM of the created nanopores. The area (A) of each of the created nanopores was determined by image processing. Next, the nanopore was approximated by a perfect circle, and the diameter (d*) thereof was determined as SQRT(4A/π). In addition, the nanopore was approximated by an ellipse, and the ellipticity (minor-axis length/major-axis length) of the ellipse was determined. The results are shown in Table 1.

**[Table 1]**

| No. | *d*, target diameter [nm] | *A*, measured area [nm²] | *d**, estimated diameter when approx. as circle [nm] | *a*/*b,* ellipticity when approx. as ellipse |
|---|---|---|---|---|
| A | 0.7 | 0.43 | 0.74 | 0.73 |
| B | 2 | 1.4 | 1.3 | 0.74 |
| C | 3 | 7.1 | 3.0 | 0.88 |
| D | 4 | 13.1 | 4.1 | 0.85 |
| E | 5 | 19.3 | 5.0 | 1.00 |
| F | 6 | 28.9 | 6.1 | 0.85 |
| G | 8 | 35.8 | 6.8 | 0.96 |
| H | 9 | 63.1 | 9.2 | 0.99 |

Thus, it was demonstrated that nanopores that very closely matched the target conditions (size and shape) were produced. The above is merely one experimental example, and it is clear that nanopores having closer values with respect to the target shapes can be produced. That is, as shown in some examples of the above table, it is possible to produce pores having target diameters and/or good ellipticity (close to a perfect circle) in nanometer-sized diameters.

### <Nanopore processing system>

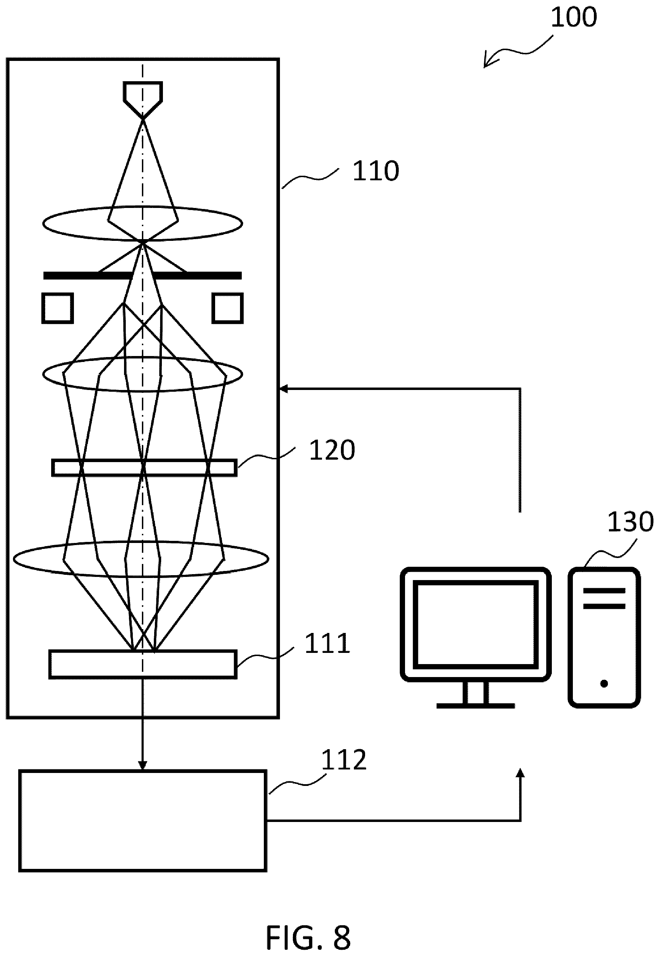

Fig. 8 schematically illustrates a nanopore processing system 100 according to an embodiment of the present disclosure. The nanopore processing system 100 includes an electron microscope body 110 including at least an optical system and a detector 111 and a computing system 130.

In general, the electron microscope body 110 is a STEM, TEM, or TEM/STEM. The electron microscope body 110 includes therein an electronic circuit unit 112 as a part or connected to the outside. The electron microscope body 110 can irradiate a sample 120 introduced therein with an electron beam probe under desired conditions. The detector 111 can receive electrons that have passed through or been diffracted from the sample 120 and convert the electrons into output signals. The detector 111 is typically a bright-field (BF) detector, a dark-field (DF) detector, a high-angle annular dark-field (HAADF) detector, an EELS detector, an EDX detector, or the like.

The electronic circuit unit 112 includes, as an example, a converter, a modulator, an amplifier, a computing unit, etc. (not illustrated) that process the signals output from the detector 111. The electronic circuit unit 112 is incorporated in many electron microscopes The electronic circuit unit 112 can output information such as obtained image data and photographing condition data to the outside.

The computing system 130 includes, for example, a central processing unit (CPU) and a memory. The computing system 130 processes image information (such as a real image of a bright-field image or a dark-field image, a diffraction image, or a Ronchigram image) of the sample 120 output from the electronic circuit unit 112 and can determine, for example, whether a thin film has changed due to electron beam irradiation, whether a pore has been formed, and the shape of a pore.

The computing system 130 transmits an instruction for the next irradiation for processing to the electron microscope body 110 on the basis of the above determination results. The computing system 130 may transmit irradiation conditions (irradiation conditions for processing) of the electron beam probe. Examples of the irradiation conditions include, but are not limited to, electron beam conditions, movement of the irradiation position (movement of the sample 120 with respect to the optical axis of the electron microscope body 110), setting of the focal position (focused irradiation, defocused irradiation, etc.), and movement profiles of the focal position (conditions such as the number of times and the time of focused irradiation that should be performed before the next observation). A driver that actually drives the electron microscope body 110 on the basis of these irradiation conditions may be configured to be incorporated into the computing system 130 or the electron microscope body 110 or may be connected to them and disposed outside (not illustrated).

Upon receiving information indicating that predetermined irradiation for processing has been finished and observed image information from the electronic circuit unit 112, the computing system 130 processes the image information, makes a determination regarding the pore, and transmits an instruction for the next irradiation for processing. This is continued until it is determined that a predetermined pore has been formed.

When the pore processing is completed, the computing system 130 notifies the user of an instruction to take out the sample 120 from the electron microscope body 110, or notifies the electron microscope body 110 of an instruction to take out the sample 120 to the outside.

The electron microscope body 110 may include a sample holder for holding a plurality of samples 120 (not illustrated). This enables, for example, a large number of nanopores to be efficiently produced by performing evacuation once.

### <Application examples>

Nanopores provided in the present disclosure can be used to measure various target substances. In some embodiments, a target substance may be caused to pass through a nanopore, and a change in an electrical signal at that time may be measured.

When a thin film having a nanopore is immersed in a conducting fluid and a voltage potential is applied between both sides thereof, an ionic current passing through the nanopore can be observed. This ionic current is determined by the effective cross-sectional area of a target substance. When a target substance passes through a nanopore, the target substance partially clogs the nanopore to change the magnitude of the current passing through the nanopore. Since the entirety or part of each target substance has a specific shape and size, the ionic current changes depending on individual passing parts. The target substance can be determined by reading this change.

In some embodiments, the target substance may be a biomolecule. In some embodiments, the target substance may be a non-biomolecule (an organic substance or an inorganic substance). In some embodiments, the target substance may be a molecule including a biomolecule that is to be measured or may be a complex of biomolecules.

The target substance may be a nucleic acid. The nucleic acid may be a deoxyribonucleic acid (DNA) or may include a DNA. The nucleic acid may be a ribonucleic acid (RNA) or may include a ribonucleic acid (RNA). A nucleotide sequence can be determined by causing a nucleic acid (DNA or RNA) to pass through a nanopore and measuring a change in the electrical signal at that time.

The biomolecule may be, for example, a protein, a virus, a bacterium, or a cell (such as an erythrocyte, a leukocyte, or an immunocyte), may include any of these, or may be selected from these.

The present disclosure also includes embodiments below.
A001 A method for processing a thin film, the method including:
   (a) providing a thin film;
   (b) irradiating the thin film with an electron beam;
   (c) observing the thin film to determine whether a pore satisfying a predetermined condition has been formed; and
   (d) if it is determined in (c) that the pore satisfying the predetermined condition has not been formed, repeating (b) and (c).
A002 A method for processing a thin film, the method including:
   (a0) providing a thin film;
   (b1) irradiating the thin film with a first electron beam to form a pore in the thin film;
   (b2) irradiating an edge of the pore with a second electron beam to change a shape of the pore;
   (c2) observing the shape of the pore; and
   (d2) if the observed shape of the pore does not satisfy a predetermined condition, repeating (b2) and (c2).
A003 A method for processing a thin film, the method including:
   (A0) providing a thin film;
   (B1) irradiating the thin film with a first electron beam (to form a pore in the thin film);
   (C1) observing the thin film to check whether a pore satisfying a predetermined condition has been formed;
   (D1) if it is determined in (C1) that the pore satisfying the predetermined condition has not been formed, repeating (B 1) and (C1);
   (B2) irradiating an edge of a pore of the thin film with a second electron beam;
   (C2) observing a shape of the pore; and
   (D2) if the shape of the observed pore does not satisfy the predetermined condition, repeating (B2) and (C2).
A011 The method according to any one of embodiments A001 to A003,
   wherein the thin film has a thickness of 10 nm or a thickness of less than 10 nm. A012 The method according to any one of embodiments A001 to A011,
   wherein the thin film is a crystalline material, and the crystalline material is a monolayer.
A015 The method according to any one of embodiments A001 to A012,
   wherein the thin film has a monolayer of a transition metal dichalcogenide or graphene.
A016 The method according to any one of embodiments A001 to A015,
   wherein the thin film is crystalline MoS₂.
A021 The method according to any one of embodiments A001 to A016,
   wherein the electron beam, the first electron beam, and/or the second electron beam has a diameter of 2 nm or less.
A022 The method according to embodiment A021,
   wherein the electron beam, the first electron beam, and/or the second electron beam has a diameter of 1 nm or less.
A031 The method according to any one of embodiments A001 to A022,
   wherein the electron beam, the first electron beam, and/or the second electron beam is a focused electron beam.
A032 The method according to embodiment A031,
   wherein irradiation with the first electron beam and/or the second electron beam includes repeatedly moving a focal point of the focused electron beam so as to pass through the thin film.
A033 The method according to embodiment A032,
   wherein (d), (d2), and/or (D2) includes acquiring an image when the focal point of the focused electron beam lies outside the thin film.
A041 The method according to any one of embodiments A001 to A033,
   wherein (d), (d2), and/or (D2) includes acquiring an image of a field of view including an irradiation region of the electron beam or the pore.
A042 The method according to embodiment A041,
   wherein the observing the shape of the pore or the observing the thin film includes measuring a size of the pore from the acquired image of the pore.
A051 The method according to any one of embodiments A001 to A042,
   wherein (b), (c), (B1), (C1), (B2), and/or (C2) is performed using a scanning transmission electron microscope (STEM).
A052 The method according to A051,
   wherein irradiation with the electron beam, the first electron beam, and/or the second electron beam is performed in a spot mode of the STEM.
B001 A program for causing a computer to execute the method according to any one of A001 to A052.
C001 A storage medium in which the program according to B001 is recorded.
D001 A system being a unit for processing a pore, the system including:
   an electron gun capable of performing spot irradiation on an introduced sample and a lens system,
      the lens system being a focusing lens,
      the lens system including an objective lens capable of repeatedly moving a focal point of the spot irradiation so as to switch between conditions of in-focus and defocus with respect to the sample;
   a bright-field detector capable of acquiring an image during the defocus; and
   a computer
      that receives output including the image during the defocus from the bright-field detector,
      that analyses the image,
      that determines a presence or absence of a pore of the sample,
      that evaluates a shape of the pore and determines, based on a result thereof, whether next spot irradiation is performed or processing is finished, and
      that determines a position of the next spot irradiation if the next spot irradiation is performed, and outputs an instruction to the lens system to perform spot irradiation on the position.
D002 The system according to D001, including an electron microscope (scanning transmission electron microscope).
E001 A thin film having a nanopore,
   wherein the nanopore has a maximum width of 10 nm or less, and
   the nanopore has an approximate ellipticity of 80% or more.
E101 A nanopore device including the thin film having the nanopore.
E111 A nucleic acid sequencer that detects a nucleic acid molecule or a nucleotide passing through the nanopore.

While preferred embodiments of the present invention have been illustrated and described herein, it will be obvious to those skilled in the art that such embodiments are provided by way of examples only. It is not intended that the invention be limited by the specific examples provided within the specification. While the invention has been described with reference to the foregoing specification, the descriptions and illustrations of the embodiments herein are not meant to be construed in a limiting sense. Numerous variations, modifications, and substitutions will now occur to those skilled in the art without departing from the invention. Furthermore, it should be understood that all aspects of the invention are not limited to the particular depictions, configurations, or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations, or equivalents. It is intended that the following claims define the scope of the invention and that methods and structures within the claims and their equivalents be covered thereby.

## Claims

1. A method for processing a thin film, the method comprising:
(a) providing a thin film;
(b) irradiating the thin film with an electron beam;
(c) observing the thin film to determine whether a pore satisfying a predetermined condition has been formed; and
(d) if it is determined in (c) that the pore satisfying the predetermined condition has not been formed, repeating (b) and (c).

2. The method according to claim 1,
wherein the thin film is a crystalline material having a thickness of 10 nm or a thickness of less than 10 nm.

3. The method according to claim 1,
wherein the thin film has a monolayer of a transition metal dichalcogenide or graphene.

4. The method according to any one of claims 1 to 3,
wherein the electron beam has a diameter of 2 nm or less.

5. The method according to claim 4,
wherein the electron beam is a focused electron beam.

6. The method according to claim 5,
wherein irradiation with the electron beam includes repeatedly moving a focal point of the focused electron beam so as to pass through the thin film.

7. The method according to claim 6,
wherein (d) includes acquiring an image when the focal point of the focused electron beam lies outside the thin film.

8. The method according to claim 1,
wherein (d) includes acquiring an image of a field of view including an irradiation region of the electron beam or the pore.

9. The method according to claim 8,
wherein the observing the thin film includes measuring a size of the pore from the acquired image of the pore.

10. The method according to claim 1,
wherein (b) and/or (c) is performed using a scanning transmission electron microscope (STEM).

11. The method according to claim 10,
wherein irradiation with the electron beam is performed in a spot mode of the STEM.

12. A program for causing a computer to execute the method according to any one of claims 1 to 11.

13. A system being a unit for processing a pore, the system comprising:
an electron gun capable of performing spot irradiation on an introduced sample and a lens system,
the lens system being a focusing lens system,
the lens system including an objective lens capable of repeatedly moving a focal point of the spot irradiation so as to switch between conditions of in-focus and defocus with respect to the sample;
a bright-field detector capable of acquiring an image during the defocus; and
a computer
that receives output including the image during the defocus from the bright-field detector,
that analyses the image,
that determines a presence or absence of a pore of the sample,
that evaluates a shape of the pore and determines, based on a result thereof, whether next spot irradiation is performed or processing is finished, and
that determines a position of the next spot irradiation if the next spot irradiation is performed, and outputs an instruction to the lens system to perform spot irradiation on the position.

14. The system according to claim 13, comprising an electron microscope (scanning transmission electron microscope).

15. A thin film having a nanopore,
wherein the nanopore has a maximum width of 10 nm or less, and
the nanopore has an approximate ellipticity of 80% or more.

16. A nanopore device comprising the thin film having the nanopore.

17. A nucleic acid sequencer that detects a nucleic acid molecule or a nucleotide passing through the nanopore.

18. A method for processing a thin film, the method comprising:
(a0) providing a thin film;
(b1) irradiating the thin film with a first electron beam to form a pore in the thin film;
(b2) irradiating an edge of the pore with a second electron beam to change a shape of the pore;
(c2) observing the shape of the pore; and
(d2) if the observed shape of the pore does not satisfy a predetermined condition, repeating (b2) and (c2).

19. A method for processing a thin film, the method comprising:
(A0) providing a thin film;
(B1) irradiating the thin film with a first electron beam (to form a pore in the thin film);
(C1) observing the thin film to check whether a pore satisfying a predetermined condition has been formed;
(D1) if it is determined in (C1) that the pore satisfying the predetermined condition has not been formed, repeating (B 1) and (C1);
(B2) irradiating an edge of a pore of the thin film with a second electron beam;
(C2) observing a shape of the pore; and
(D2) if the shape of the observed pore does not satisfy the predetermined condition, repeating (B2) and (C2).

## Amended claims

### Amended claims under Art. 19.1 PCT

1. A method for processing a thin film, the method comprising:
(a) providing a thin film;
(b) irradiating the thin film with a focused electron beam, the irradiating with the focused electron beam including repeatedly moving a focal point of the focused electron beam so as to pass through the thin film;
(c) observing the thin film to determine whether a pore satisfying a predetermined condition has been formed; and
(d) if it is determined in (c) that the pore satisfying the predetermined condition has not been formed, repeating (b) and (c).

2. The method according to claim 1,
wherein the thin film is a crystalline material having a thickness of 10 nm or a thickness of less than 10 nm.

3. The method according to claim 1,
wherein the thin film has a monolayer of a transition metal dichalcogenide or graphene.

4. The method according to claim 1,
wherein the focused electron beam has a diameter of 2 nm or less.

5. The method according to claim 4,
wherein the electron beam is a focused electron beam.

6. The method according to claim 5,
wherein irradiation with the electron beam includes repeatedly moving a focal point of the focused electron beam so as to pass through the thin film.

7. The method according to claim 1,
wherein (d) includes acquiring an image when the focal point of the focused electron beam lies outside the thin film.

8. The method according to claim 1,
wherein (d) includes acquiring an image of a field of view including an irradiation region of the electron beam or the pore.

9. The method according to claim 8,
wherein the observing the thin film includes measuring a size of the pore from the acquired image of the pore.

10. The method according to claim 1,
wherein (b) and/or (c) is performed using a scanning transmission electron microscope (STEM).

11. The method according to claim 10,
wherein irradiation with the electron beam is performed in a spot mode of the STEM.

12. A program for causing a computer to execute the method according to any one of claims 1 to 11.

13. A system being a unit for processing a pore, the system comprising:
an electron gun capable of performing spot irradiation on an introduced sample and a lens system,
the lens system being a focusing lens system,
the lens system including an objective lens capable of repeatedly moving a focal point of the spot irradiation so as to switch between conditions of in-focus and defocus with respect to the sample;
a bright-field detector capable of acquiring an image during the defocus; and
a computer
that receives output including the image during the defocus from the bright-field detector,
that analyses the image,
that determines a presence or absence of a pore of the sample,
that evaluates a shape of the pore and determines, based on a result thereof, whether next spot irradiation is performed or processing is finished, and
that determines a position of the next spot irradiation if the next spot irradiation is performed, and outputs an instruction to the lens system to perform spot irradiation on the position.

14. The system according to claim 13, comprising a scanning transmission electron microscope.

15. A thin film having a nanopore,
wherein the nanopore has a maximum width of 10 nm or less, and
the nanopore has an approximate ellipticity of 80% or more.

16. A nanopore device comprising the thin film having a nanopore processed by the method according to any one of claims 1 to 14.

17. A nucleic acid sequencer that detects a nucleic acid molecule or a nucleotide passing through a nanopore processed by the method according to any one of claims 1 to 8.

18. A method for processing a thin film, the method comprising:
(a0) providing a thin film;
(b1) irradiating the thin film with a first focused electron beam to form a pore in the thin film, the irradiating with the first focused electron beam including repeatedly moving a focal point of the first focused electron beam so as to pass through the thin film;
(b2) irradiating an edge of the pore with a second focused electron beam to change a shape of the pore, the irradiating with the second focused electron beam including repeatedly moving a focal point of the second focused electron beam so as to pass through the thin film;
(c2) observing the shape of the pore; and
(d2) if the observed shape of the pore does not satisfy a predetermined condition, repeating (b2) and (c2).

19. A method for processing a thin film, the method comprising:
(A0) providing a thin film;
(B1) irradiating the thin film with a first focused electron beam to form a pore in the thin film, the irradiating with the first focused electron beam including repeatedly moving a focal point of the first focused electron beam so as to pass through the thin film;
(C1) observing the thin film to check whether a pore satisfying a predetermined condition has been formed;
(D1) if it is determined in (C1) that the pore satisfying the predetermined condition has not been formed, repeating (B 1) and (C1);
(B2) irradiating an edge of the pore of the thin film with a second focused electron beam, the irradiating with the second focused electron beam including repeatedly moving a focal point of the second focused electron beam so as to pass through the thin film;
(C2) observing a shape of the pore; and
(D2) if the shape of the observed pore does not satisfy the predetermined condition, repeating (B2) and (C2).

20. The method according to claim 7,
wherein the acquiring the image includes acquiring a Ronchigram.

Statement under Art. 19.1 PCT
1. The applicant has amended claims 1, 18, and 19 to incorporate claims 5 and 6 before the amendment. Accordingly, the wording of claim 4 has been amended, and claims 5 and 6 have been canceled. Amendments for correcting errors have been made to claims 16 and 17. New claim 20, which is dependent on claim 7, has been added. New claim 20 is based on the description of paragraphs 0052 to 0057 in the specification.
